# EUROPÄISCHE PATENTSCHRIFT

(11) **EP 2 869 912 B1**
(45) Veröffentlichungstag und Bekanntmachung des Hinweises auf die Patenterteilung: **29.11.2017**
(21) Anmeldenummer: 13730511.6
(22) Anmeldetag: 12.06.2013
(51) Int. Cl.: B01D 69/08, B29C 47/20

(54) **SCHLAUCHMATTE UND VERFAHREN ZUM HERSTELLEN EINER DERARTIGEN SCHLAUCHMATTE**
TUBE MAT AND METHOD FOR PRODUCING SAID TUBE MAT
MAT TUBULAIRE ET PROCÉDÉ DE PRODUCTION D'UN MAT TUBULAIRE DE CE TYPE

(30) Priorität: 04.07.2012 DE 102012211617
(43) Veröffentlichungstag der Anmeldung: 13.05.2015
(60) Teilanmeldung: 17196737.5
(73) Patentinhaber: Raumedic AG, 95213 Münchberg (DE)
(72) Erfinder: ERHARD, Dominik, 95326 Kulmbach (DE); STÖCKER, Martin, 95233 Helmbrechts (DE)
(74) Vertreter: Rau, Schneck & Hübner Patentanwälte Rechtsanwälte PartGmbB
(86) Internationale Anmeldenummer: PCT/EP2013/062087
(87) Internationale Veröffentlichungsnummer: WO 2014/005809

(56) Entgegenhaltungen:
- WO-A1-2012/040778
- GB-A- 2 268 635
- US-A- 4 332 752

## Beschreibung

Die Erfindung betrifft eine Schlauchmatte, ein Verfahren zum Herstellen einer derartigen Schlauchmatte sowie ein Werkzeug zum Extrudieren einer derartigen Schlauchmatte.

Aus der DE 28 38 659 C2 ist eine Schlauchanordnung für eine Viertelgemelksmaschine bekannt. Die Schlauchanordnung weist vier parallel zueinander laufende Schlauchleitungen auf, die an den Ecken eines Rhombus liegen. Die Schlauchanordnung ist keine Schlauchmatte.

Die DE 35 87 787 T2 offenbart ein Verfahren zur Herstellung von Hohlfasern mittels Schmelzspinnen. Dazu sind speziell ausgeführte Spinndüsen erforderlich. Aus Kunststoff hergestellte Hohlfasern werden mit einer Spinnflüssigkeit benetzt. Ein derartiges Verfahren ist komplex.

Aus der DE 18 08 271 A1 sind Schläuche bekannt, die aus Stabilitätsgründen Verstärkungselemente wie Rippen aufweisen können. Aus der DE 43 04 246 A1 ist ein Verfahren zur Herstellung eines Schlauchs bekannt. Eine Schlauchmatte ist aus der DE 43 04 246 A1 nicht bekannt.

Die DE 27 04 678 A1 offenbart ein Verfahren zur Extrusion eines Kunststoffnetzes.

Bei einer Operation am offenen Herzen wird Blut eines Patienten in einer Herz-Lungen-Maschine extrakorporal mit Sauerstoff versorgt und gleichzeitig Kohlendioxid entfernt. Es handelt sich um die sogenannte extrakorporale Zirkulation (ECC). Eine ähnliche Blutbehandlung kommt auch bei einer extrakorporalen Membranoxygenierung (ECMO) zur pulmonalen Unterstützung eines Lungenpatienten zum Einsatz. In beiden Fällen werden Hohlfaserbündel verwendet, wobei Sauerstoff und Blut jeweils getrennt voneinander durch die von den Hohlfasern gebildeten Lumen bzw. durch die Zwischenräume der Hohlfasern geleitet werden.

Ein derartiges Hohlfaserbündel ist aus der DE 10 2010 000 820 A1 bekannt. Mehrere Schlauchabschnitte werden mit ihren Schlauchlängsachsen parallel zueinander angeordnet und in einer Richtung senkrecht zu den Schlauchlängsachsen mittels mindestens einer Wirknaht miteinander verwirkt.

Die WO 2012/040 778 A1 offenbart einen Solarkollektor zum Erwärmen und Fördern von Flüssigkeiten. Der Solarkollektor weist eine Schlauchmatte auf, deren Einzelschläuche über ein Verteilerrohr verbunden sind.

Weitere Schlauchmatten sind bekannt aus der GB 2 268 635 A und aus der US 4,332,752.

Es ist eine Aufgabe der vorliegenden Erfindung, eine Schlauchmatte derart weiterzuentwickeln, dass sie vereinfacht hergestellt werden kann.

Diese Aufgabe ist erfindungsgemäß gelöst durch eine Schlauchmatte mit den im Anspruch 1 angegebenen Merkmalen.

Die erfindungsgemäße Schlauchmatte ist einstückig hergestellt. Insbesondere ist es nicht erforderlich, mehrere Schlauchabschnitte einzeln herzustellen, diese definiert zueinander anzuordnen und anschließend miteinander zu verwirken. Durch die einstückige Herstellung der Schlauchmatte sind Schlauchabschnitte automatisch zueinander definiert angeordnet und miteinander verbunden. Insbesondere sind die Schlauchabschnitte mit ihren Schlauchlängsachsen jeweils parallel zueinander angeordnet. Die Schlauchabschnitte sind jeweils durch einen Stegabschnitt miteinander verbunden. Die Herstellung der erfindungsgemäßen Schlauchmatte ist vereinfacht. Insbesondere ist die Anzahl der Verfahrensschritte reduziert. Die Anzahl der Einzelteile ist reduziert, so dass der Montageaufwand für die Schlauchmatte entfällt. Der Montageaufwand für die Herstellung eines Wärmetauschers insgesamt ist dadurch reduziert. Der Zeitaufwand und der Kostenaufwand für die Herstellung der erfindungsgemäßen Schlauchmatte sind reduziert. Insbesondere kann die Schlauchmatte durch Extrusion einstückig hergestellt werden. Das Risiko von Verunreinigungen bei der Herstellung der Schlauchmatte ist minimiert, da insbesondere ein Verwirken mit Wirkfäden und somit ein Einbringen von Staub und/oder Fasernabrieb ausgeschlossen ist. Die Schlauchmatte weist hinsichtlich ihrer Anwendung eine erhöhte Zuverlässigkeit auf. Die Ausschussrate bei der Herstellung der Schlauchmatte ist reduziert. Das Risiko, dass eine unbemerkte Verunreinigung der Schlauchmatte zu einer Gesundheitsgefährdung eines Patienten führt, ist reduziert. Derartige Schlauchmatten können sowohl in einem Oxygenator für die extrakorporale Zirkulation (ECC) als auch für die extrakorporale Membranoxigenierung (ECMO) eingesetzt werden. Die Schlauchmatte eignet sich grundsätzlich auch zur Verwendung im Bereich der pharmazeutischen Industrie für sogenannte Bioreaktoren. Beispielsweise sind Fermenter bekannt, bei welchen eine Bakterienkultur in Lösung durch Hohlfasern mit Sauerstoff versorgt wird. Derartige Hohlfasern können durch die Schlauchmatte abgebildet werden. Es ist auch bekannt, Oxygenierungsfasern bei der Gewebezüchtung, dem sogenannten Tissue Engineering, einzusetzen. Die Schlauchmatten können auch als Wärmetauschermatten beispielsweise in einer Fußbodenheizung, einer Wandheizung und/oder entsprechenden Kühlelementen Verwendung finden. Die erfindungsgemäße Schlauchmatte ist als Matte in einer Ausgangsanordnung im Wesentlichen flächig ausgeführt. Das bedeutet, dass die Längsachsen der Schlauchabschnitte in einer Mattenebene anordenbar sind. Es ist möglich, die Schlauchmatte einzeln oder mit mindestens einer weiteren Schlauchmatte zu einem Schlauchmattenbündel einzurollen, sodass insbesondere ein im Wesentlichen zylinderförmiges Schlauchmattenbündel resultiert.

Eine Schlauchmatte mit mindestens einem Abstandshalter, der eine senkrecht zu einer Mattenebene orientierte Dicke aufweist, die größer ist als ein Außendurchmesser eines Schlauchabschnitts ermöglicht eine vereinfachte Herstellung eines Schlauchbündels aus mehreren Schlauchmatten. Durch den mindestens einen Abstandshalter können die Schlauchabschnitte einer Schlauchmatte in einem definierten Abstand zueinander angeordnet werden. Insbesondere werden mehrere Schlauchmatten zu einem Schlauchbündel aufgerollt bzw. aufgewickelt.

Eine Schlauchmatte nach Anspruch 2 ermöglicht eine flexible Verbindung der Schlauchmatten zu einem Schlauchmattenbündel.

Eine Schlauchmatte nach einem der Ansprüche 3 oder 4 ermöglicht eine Verbindung einzelner Schlauchmatten miteinander mit einer erhöhten Festigkeit. Einzelne Abstandshalter können insbesondere in korrespondierende Zwischenräume einer anderen Schlauchmatte bei der Verbindung der einzelnen Schlauchmatten miteinander eingreifen. Der Materialeinsatz für die Herstellung der Abstandshalter entlang der Schlauchlängsachse gegenüber einem durchgängigen, stegförmigen Abstandshalter ist reduziert.

Eine Schlauchmatte nach Anspruch 5 weist ein erhöhtes Flächenträgheitsmoment bezüglich einer Biegung um eine zu den Schlauchlängsachsen quer gerichteten Richtung auf. Die Schlauchmatte ist stabil ausgeführt. Insbesondere sind die Abstandshalter als durchgängige Stege ausgeführt. Derartige Stege sind vereinfacht herstellbar.

Eine Schlauchmatte nach Anspruch 6 ermöglicht eine verbesserte Verbindung mit einer anderen Schlauchmatte zu einem Schlauchmattenbündel.

Eine Schlauchmatte nach Anspruch 7 ermöglicht Materialeinsparung bei der Herstellung. Zusätzlich weist eine derartige Schlauchmatte eine erhöhte Diffusionsoberfläche auf.

Eine Schlauchmatte nach Anspruch 8 ist vereinfacht herstellbar. Dadurch, dass mehrere Schlauchabschnitte gleichzeitig als einstückiges Bauteil in Form der Schlauchmatte extrudiert werden, kann die Matte insgesamt besser beim Extrudieren abgezogen werden. Die Gefahr eines Reißens einzelner Silikonschlauchabschnitte ist reduziert. Die Herstellung der Schlauchmatte ist zuverlässig durchführbar. Eine derartige Schlauchmatte ist insbesondere für den Einsatz in einem Oxygenatormodul bei der ECC geeignet. Alternativ ist es auch möglich, Polyurethan oder andere thermoplastische Polymere für die Herstellung der Schlauchmatte zu verwenden. In diesem Fall kann die Schlauchmatte auch für Wärmetauscher im Allgemeinen verwendet werden.

Es ist eine weitere Aufgabe der vorliegenden Erfindung, ein Verfahren zum Herstellen einer Schlauchmatte zu vereinfachen.

Diese Aufgabe wird durch ein Verfahren mit den im Anspruch 9 angegebenen Merkmalen gelöst. Bei einem Verfahren zum Herstellen einer Schlauchmatte wird zunächst ein Werkzeug bereitgestellt, mit dem eine Schlauchmatte entlang einer Extrusionsrichtung extrudiert werden kann. Anschließend erfolgt ein Extrudieren der Schlauchmatte, die mehrere entlang der Extrusionsrichtung angeordnete Schlauchabschnitte und mindestens einen, zwei Schlauchabschnitte verbindenden Stegabschnitt aufweist. Dadurch ist es möglich, mehrere Schlauchabschnitte, die jeweils durch Stegabschnitte miteinander verbunden sind, einstückig in einem Verfahrensschritt durch Extrudieren herzustellen. Ein derartiges Verfahren ist wirtschaftlich. Dadurch, dass mehrere Schlauchabschnitte durch die Stegabschnitte miteinander verbunden sind, entsteht eine Schlauchmatte mit einer verbesserten Strukturstabilität. Insbesondere kann die Schlauchmatte besser aus dem Extrusionswerkzeug abgezogen werden. Insbesondere ist auch die Handhabung für möglicherweise nachgelagerte Verfahrensschritte verbessert. Insbesondere kann eine derart hergestellte Schlauchmatte besser abgelegt und/oder gewickelt werden. Beispielsweise können mehrere Schlauchmatten zu einem Schlauchbündel verbunden werden, indem insbesondere ein Schlauchabschnitt einer ersten Schlauchmatte auf einem Stegabschnitt einer zweiten Schlauchmatte angeordnet und diese anschließend zu einem Schlauchbündel durch Rollen verbunden werden.

Es ist außerdem eine weitere, nicht erfindungsgemäße Aufgabe der vorliegenden Erfindung, ein Werkzeug zum Extrudieren einer Schlauchmatte bereitzustellen.

Ein derartiges Werkzeug dient zum Extrudieren einer Schlauchmatte entlang einer Extrusionsrichtung. Das Werkzeug weist eine Extrusionsdüse auf, die mit einer Extrusionskammer verbindbar ist. In der Extrusionskammer kann die zu extrudierende Kunststoffschmelze bevorratet sein. Die Extrusionsdüse weist eine Mattenkavität auf, die mehrere, jeweils eine Längsachse aufweisende und parallel zur Extrusionsrichtung angeordnete Schlauchkavitäten sowie mindestens eine, jeweils zwei benachbarte Schlauchkavitäten verbindende Stegkavitäten umfasst. In jeder Schlauchkavität ist ein Dorn angeordnet, so dass zwischen einer Außenwand des Dorns und einer Innenwand der Schlauchkavität ein im Wesentlichen ringringförmig ausgeführter Spalt gebildet wird. Da mehrere Schlauchkavitäten vorgesehen sind, sind auch mehrere Dorne von dem Werkzeug umfasst.

Ausführungsbeispiele der Erfindung werden nachfolgend anhand der Zeichnung näher erläutert. In dieser zeigen:
- Fig. 1: eine Draufsicht auf eine nicht-erfindungsgemäße Schlauchmatte,
- Fig. 2: eine Seitenansicht der Schlauchmatte in Fig. 1,
- Fig. 3: eine Schnittdarstellung gemäß Linie III-III in Fig. 1,
- Fig. 4: eine Fig. 1 entsprechende Draufsicht einer Schlauchmatte gemäß einer nicht-erfindungsgemäßen Ausführungsform,
- Fig. 5: eine Fig. 3 entsprechende Schnittdarstellung einer nichterfindungsgemäßen Ausführungsform einer Schlauchmatte,
- Fig. 6, 7: Fig. 2 entsprechende Seitenansichten nichterfindungsgemäßen Ausführungsformen einer Schlauchmatte,
- Fig. 8: eine Fig. 3 entsprechende Schnittdarstellung einer erfindungsgemäßen Ausführungsform einer Schlauchmatte,
- Fig. 9, 10: schematische Darstellungen zur Anordnung mehrerer Schlauchmatten für die Herstellung eines Schlauchbündels,
- Fig. 11: eine schematische Darstellung einer Anlage mit einem Werkzeug zum Extrudieren einer Schlauchmatte,
- Fig. 12: eine Schnittdarstellung gemäß Linie XII-XII in Fig. 11 und
- Fig. 13: eine Fig. 12 entsprechende Schnittdarstellung eines Werkzeugs gemäß einer weiteren Ausführungsform.

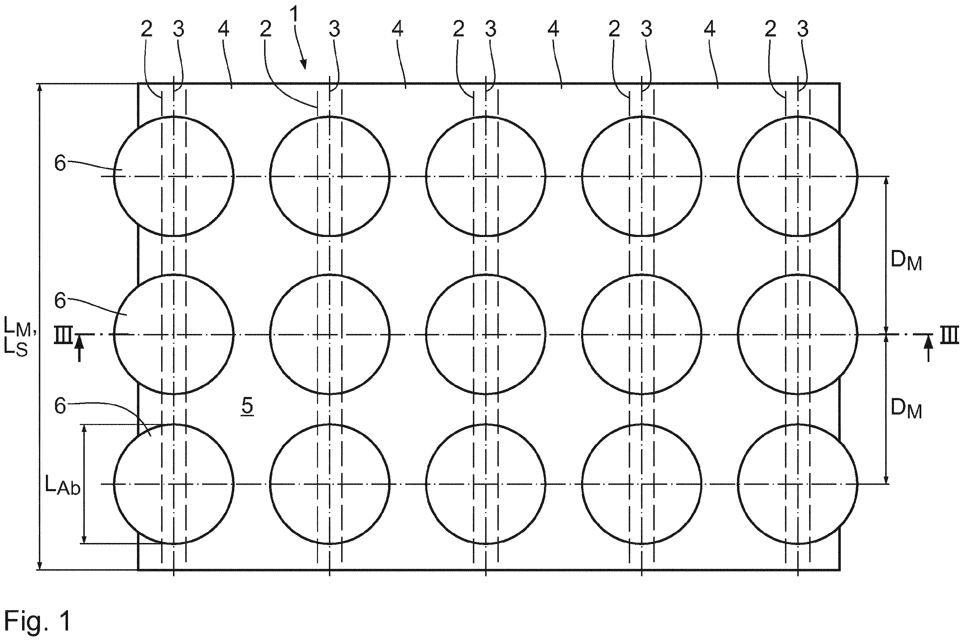

Eine in Fig. 1 bis 3 dargestellte Schlauchmatte 1 weist fünf Schlauchabschnitte 2 auf. Die Schlauchabschnitte 2 sind jeweils mit ihrer Schlauchlängsachse 3 parallel zueinander orientiert. Jeweils zwei Schlauchabschnitte 2 sind jeweils durch einen Stegabschnitt 4 miteinander verbunden. Gemäß dem gezeigten Ausführungsbeispiel sind also fünf Schlauchabschnitte 2 und vier Stegabschnitte 4 vorgesehen.

Jeder Schlauchabschnitt 2 weist eine Schlauchwand mit einer Schlauchwanddicke d_{w} im Bereich von 10 µm bis 200 µm auf, bevorzugt zwischen 10 µm und 50 µm. Ein Innendurchmesser dᵢ eines Schlauchabschnitts 2 beträgt zwischen 10 µm und 1000 µm, bevorzugt zwischen 10 µm und 500 µm. Entsprechend beträgt ein Außendurchmesser dₐ eines Schlauchabschnitts zwischen 30 µm und 1400 µm.

Der Stegabschnitt 4 weist eine Dicke d_{S} von etwa 10 µm bis 200 µm auf. Die Dicke d_{S} des Stegabschnitts 4 ist senkrecht zu einer Mattenebene 5 orientiert. Die Mattenebene 5 entspricht der Zeichenebene in Fig. 1.

Es sind auch mehr oder weniger als die in Fig. 1 bis 3 dargestellten fünf Schlauchabschnitte 2 pro Schlauchmatte 1 möglich. Die Anzahl, die Größe der Schlauchabschnitte 2 sowie die Anzahl und die Größe der Stegabschnitte 4 richten sich nach dem Verwendungszweck der Schlauchmatte 1. Insbesondere beträgt die Anzahl der Schlauchabschnitte 2 einer Schlauchmatte 1 mehr als 10.

Die Schlauchmatte 1 weist eine Länge L_{M} auf, die identisch ist mit einer Länge L_{S} der einzelnen Schlauchabschnitte 2. Die Länge L_{S} der Schlauchabschnitte 2 ist parallel zur Schlauchlängsachse 3 orientiert. Weiterhin weist die Schlauchmatte 1 eine Breite B_{M} auf.

Die Länge L_{M} der Schlauchmatte 1 beträgt insbesondere zwischen 5 cm und 30 cm in Abhängigkeit des Verwendungszwecks des Oxygenators. Beispielsweise beträgt die Länge L_{M} 5 cm bei einer Schlauchmatte 1 für einen Oxygenator eines Frühgeborenen oder beispielsweise 30 cm für einen Oxygenator einer erwachsenen Person. Bei der Herstellung ist die Länge L_{M} der Schlauchmatte 1 nicht limitiert. Insbesondere dann, wenn die Schlauchmatte 1 wie nachfolgend noch beschrieben durch Extrusion hergestellt wird, kann eine beliebige Länge L_{M} bereitgestellt werden. Es ist möglich, die Schlauchmatte 1 als Bundware zur Verfügung zu stellen, so dass zu einem späteren Zeitpunkt Schlauchmatten 1 mit einer vorgegebenen Länge L_{M} oder mit verschiedenen Längen zugeschnitten werden können. Die Breite B_{M} beträgt vorzugsweise zwischen 2 cm und 50 cm. Die Breite B_{M} ist insbesondere durch die Dimensionen der Schlauchabschnitte 2 und durch ein Werkzeug zur Herstellung der Schlauchmatte 1 mitbestimmt.

Die Schlauchmatte 1 ist einstückig aus Silikonkautschuk hergestellt. Die Schlauchmatte 1 weist ein einheitliches, homogenes Material auf. Die Schlauchmatte 1 weist homogene Materialeigenschaften auf. An den Schlauchabschnitten 2 sind jeweils entlang der Schlauchlängsachse 3 drei Abstandshalter 6 vorgesehen. Die Abstandshalter 6 sind als kugelförmige Verdickungen ausgebildet. Die Abstandshalter 6 sind jeweils aus Silikonkautschuk einstückig mit der Schlauchmatte 1 ausgeführt. Die Abstandshalter 6 weisen eine senkrecht zur Schlauchlängsachse 3 orientierte runde Querschnittsfläche auf. Die Abstandshalter 6 weisen eine Dicke d_{Ab} auf, die senkrecht zu der Mattenebene 5 orientiert ist und etwa 40 µm bis 2000 µm aufweist.

Die Schlauchmatte 1 ist durch Extrusion hergestellt. Es ist denkbar, die Schlauchmatte 1 mittels Koextrusion herzustellen und insbesondere die Schlauchabschnitte 2 und die Stegabschnitte 4 aus einem ersten Material und die Abstandshalter 6 aus einem zweiten, davon verschiedenen Material durch Koextrusion herzustellen.

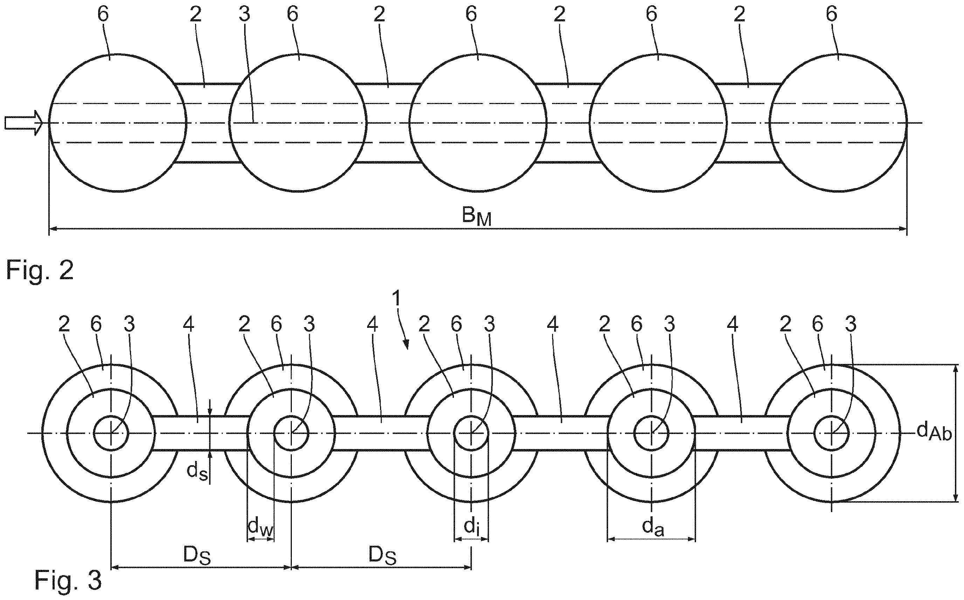

Gemäß dem gezeigten Ausführungsbeispiel ist eine Länge L_{Ab} der Abstandshalter 6 entlang der Schlauchlängsachse 3 identisch mit der Dicke d_{Ab}. Insbesondere ist die Länge L_{Ab} kleiner als die Länge L_{S} des Schlauchabschnitts 2. Die Dicke d_{Ab} des Abstandshalters 6 ist größer als der Außendurchmesser dₐ des Schlauchabschnitts 2. Die in Fig. 3 dargestellte typische Struktur der Schlauchmatte 1 entspricht der einer Perlenkette. Bei der Ausführungsform der Schlauchmatte 1 in den Fig. 1 bis 3 sind die Abstandshalter 6 entlang der Schlauchabschnitte 2 angeordnet. Ein Mittenabstand D_{M} der Abstandshalter 6 entlang der Schlauchlängsachse 3 ist identisch. Die Abstandshalter 6 sind entlang der Schlauchabschnitte 2 regelmäßig, d. h. mit gleichbleibenden Mittenabstand D_{M}, angeordnet. Die Schlauchabschnitte 2 sind in einer Richtung senkrecht zu den Schlauchlängsachsen 3 regelmäßig, d. h. mit identischen Schlauchabschnittsabständen D_{S} angeordnet. In dem Ausführungsbeispiel sind die Abstandshalter 6 in einem regelmäßigen, rechteckförmigen Raster angeordnet. Ein Rasterabstand entlang der Schlauchlängsachse 3 ist der Mittenabstand D_{M}. Ein Rasterabstand in einer zu den Schlauchlängsachsen 3 senkrecht orientierten Richtung ist der Schlauchabschnittsabstand D_{S}. Es ist auch denkbar, dass die Abstände D_{M} und/oder D_{S} bei einer Schlauchmatte 1 variieren. Die Abstandshalter 6 sind zwischen benachbarten Schlauchabschnitten 2 senkrecht zueinander angeordnet, d. h. eine gedachte Verbindungslinie von zwei Abstandshaltern 6 benachbarter Schlauchabschnitte 2 ist senkrecht zu den Schlauchlängsachsen 3 orientiert wie beispielsweise die Schnittlinie III-III. Die daraus resultierenden, charakteristischen Perlenkettenstrukturen sind entlang der Schlauchlängsachse 3 identisch.

Gemäß einer weiteren nicht dargestellten Ausführungsform einer Schlauchmatte 1 können die Abstandshalter 6 jeweils entlang eines Schlauchabschnitts 2 angeordnet sein, wobei die Abstandshalter 6 zweier benachbarter Schlauchabschnitte 2 entlang der Schlauchlängsachsen 3 versetzt zueinander angeordnet sind. Insbesondere ist es denkbar, den Schlauchabschnittsabstand D_{S} und den Mittabstand D_{M} derart zu wählen, dass sämtliche Abstandshalter 6 der Schlauchmatte 1 mit gleichem Abstand paarweise zueinander angeordnet sind. Insbesondere können die Abstandshalter 6 derart angeordnet sein, dass drei benachbarte Abstandshalter 6 die Eckpunkte eines gleichseitigen Dreiecks bilden. Dadurch ist es möglich, eine Schlauchmatte 1 mit einer dichtesten Flächenanordnung der Abstandshalter zu ermöglichen. Dadurch ist es möglich, eine höhere Flächendichte der Abstandshalter 6 zu ermöglichen, also die Anzahl der Abstandshalter 6 pro Fläche zu erhöhen.

Es ist auch denkbar, die Perlenkettenstruktur entlang der Schlauchlängsachsen 3 veränderlich auszuführen.

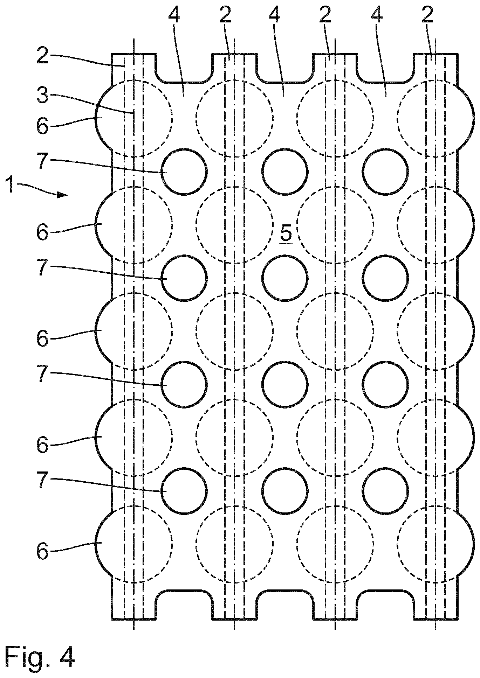

Fig. 4 zeigt eine weitere Ausführung einer Schlauchmatte 1. Komponenten, die denjenigen entsprechen, die vorstehend unter Bezugnahme auf die Fig. 1 bis 3 erläutert wurden, tragen die gleichen Bezugsziffern und werden nicht nochmals im Einzelnen diskutiert.

Die Schlauchmatte 1 nach Fig. 4 entspricht im Wesentlichen der Schlauchmatte 1 gemäß Fig. 1, wobei vier Schlauchabschnitte 2 und drei Stegabschnitte 4 vorgesehen sind. Entlang der Schlauchlängsachse 3 sind an jedem Schlauchabschnitt 2 fünf Abstandshalter 6 vorgesehen. Wesentlicher Unterschied der Schlauchmatte 1 gemäß Fig. 4 gegenüber der Schlauchmatte 1 in Fig. 1 sind mehrere Ausnehmungen 7. Die Ausnehmungen 7 weisen eine in der Mattenebene 5 kreisförmige Kontur, ovale Kontur oder rechteckige Kontur mit abgerundeten Ecken auf. Die Ausnehmungen 7 sind in einer Richtung parallel zur Schlauchlängsachse 3 zwischen zwei benachbarten Abstandshaltern 6 und in einer Richtung senkrecht zu den Schlauchlängsachsen 3 zwischen zwei benachbarten Schlauchabschnitten 2 angeordnet. Die Ausnehmungen 7 durchdringen die Stegabschnitte 4 vollständig. Die Ausnehmungen 7 sind Löcher.

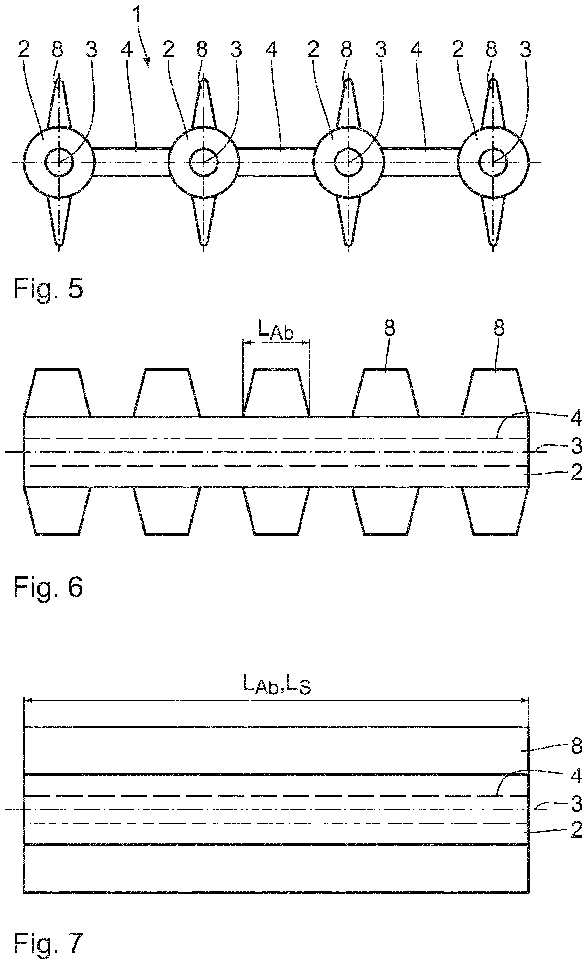

Fig. 5 bis 7 zeigen weitere Ausführungen einer Schlauchmatte 1. Komponenten, die denjenigen entsprechen, die vorstehend unter Bezugnahme auf die Fig. 1 bis 4 erläutert wurden, tragen die gleichen Bezugsziffern und werden nicht nochmals im Einzelnen diskutiert.

Die Schlauchmatte 1 gemäß Fig. 5 bis 7 unterscheidet sich im Wesentlichen von den vorangegangenen Schlauchmatten durch die Ausgestaltung der Abstandshalter 6. Die Abstandshalter 8 weisen eine senkrecht zur Schlauchlängsachse 3 orientierte Querschnittsfläche mit einer sternförmigen Kontur auf. Die Abstandshalter 8 können entsprechend den Abstandshaltern 6 eine entlang der Schlauchlängsachse 3 reduzierte Länge L_{Ab} aufweisen (Fig. 6). Es ist auch möglich, dass genau ein Abstandshalter 8 entlang der Schlauchlängsachse 3 vorgesehen ist. In diesem Fall weist der Abstandshalter 6 eine Länge L_{Ab} auf, die identisch ist mit der Länge L_{S} des Schlauchabschnitts 2 (Fig. 7).

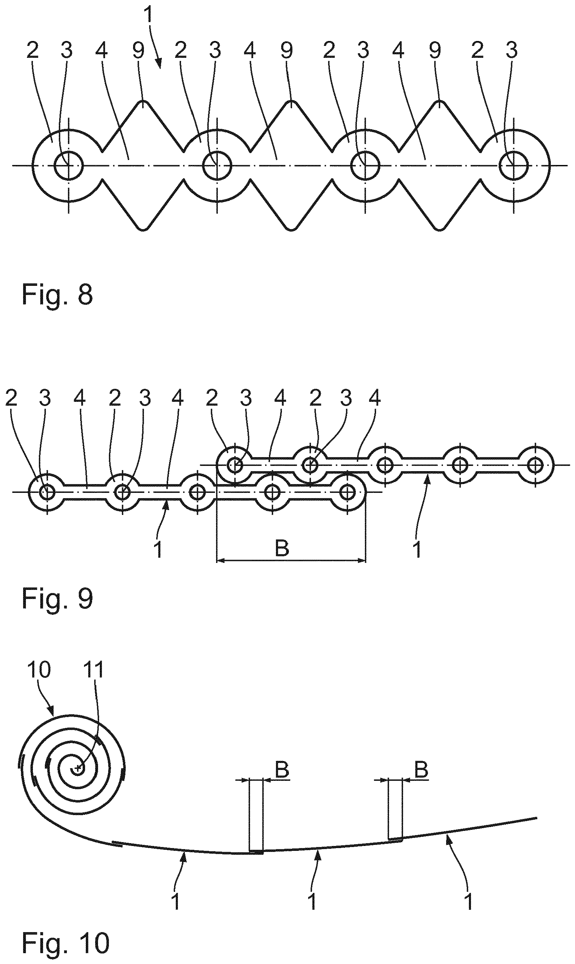

Fig. 8 zeigt eine weitere Ausführung einer Schlauchmatte 1. Komponenten, die denjenigen entsprechen, die vorstehend unter Bezugnahme auf die Fig. 1 bis 7 erläutert wurden, tragen die gleichen Bezugsziffern und werden nicht nochmals im Einzelnen diskutiert.

Die Schlauchmatte 1 unterscheidet sich von den vorhergehenden Schlauchmatten dadurch, dass die Abstandshalter 9 an den Stegabschnitten 4 angeordnet sind. Insbesondere sind die Schlauchabschnitte 2 frei von Abstandshaltern. Die Abstandshalter 9 sind ähnlich wie die Abstandshalter 8 gemäß der Schlauchmatte 1 in Fig. 5 sternförmig ausgeführt.

Gemäß einer weiteren, nicht in den Figuren dargestellten Ausführungsform ist es möglich, mehrere, verschieden geformte Abstandshalter an einer Schlauchmatte vorzusehen. Es ist möglich, die Abstandshalter sowohl im Bereich der Schlauchabschnitte als auch im Bereich der Stegabschnitte vorzusehen. Die Abstandshalter können entweder eine reduzierte Länge L_{Ab} und/oder eine Länge L_{Ab} aufweisen, die im Wesentlichen der Länge L_{S} des Schlauchabschnitts 2 entspricht.

Im Folgenden wird unter Bezugnahme auf die Fig. 9 und 10 ein Verfahren zur Herstellung eines Schlauchbündels 10 näher erläutert. Das Schlauchbündel 10 weist eine Bündellängsachse 11 auf. Das Schlauchbündel 10 ist rollenförmig ausgeführt und weist einen senkrecht zur Bündellängsachse 11 orientierten kreisförmigen Querschnitt auf. Die Bündellängsachse 11 ist parallel zu den Schlauchlängsachsen 3 orientiert.

Zur Herstellung des Schlauchbündels 10 werden mehrere Schlauchmatten 1 miteinander verbunden und anschließend zu dem Bündel 10 aufgerollt. Die Anzahl der Schlauchmatten 1, die zu einem Schlauchbündel 10 gerollt werden, hängt von der jeweiligen Breite B_{M} der Schlauchmatten 1 und dem Einsatzzweck des damit hergestellten Schlauchbündels 10 ab. Die einzelnen Schlauchmatten 1 sind in Fig. 10 schematisch dargestellt. Insbesondere ist die exakte Querschnittskontur einer Schlauchmatte, die sich aus den durch die Stegabschnitte miteinander verbundenen Schlauchabschnitte 2 ergibt, nicht im Einzelnen dargestellt. Diese Kontur ist in Fig. 9 dargestellt. Daraus geht hervor, dass an einer ersten, in Fig. 9 unten, links dargestellten Schlauchmatte 1 eine weitere, zweite Schlauchmatte 1 angeordnet wird. Die weitere Schlauchmatte 1 ist in Fig. 9 rechts, oben dargestellt. Aus Gründen der einfacheren Darstellung sind die Schlauchmatten 1 in Fig. 9 ohne Abstandshalter dargestellt. Es ist selbstverständlich möglich, dass an den Schlauchmatten 1 gemäß Fig. 9 und 10 Abstandshalter, wie vorstehend beschrieben, vorgesehen sein können. Die Schlauchmatten 1 sind in einer senkrecht zu den Schlauchlängsachsen 3 orientierten Breitenrichtung der Schlauchmatten 1 versetzt zueinander angeordnet. Das bedeutet, dass die Schlauchabschnitte 2 der einen Schlauchmatte 1 an einem Stegabschnitt 4 der anderen Schlauchmatte 1 anliegen oder zumindest dort angeordnet werden.

Das bedeutet also, dass die Schlauchmatten 1 wechselweise ineinander eingreifen. Die Schlauchmatten 1 sind mechanisch, insbesondere durch eine Art Formschluss, aneinander gehalten. Dadurch ergibt sich eine automatische Fixierung der Schlauchmatten 1 aneinander. Ein aktives Befestigen, beispielsweise durch Kleben der einzelnen Schlauchmatten miteinander, ist nicht erforderlich.

Die Schlauchmatten 1 sind in einem Überlappungsbereich B überlappend angeordnet. Der Überlappungsbereich B ist gemäß dem gezeigten Ausführungsbeispiel derart ausgeführt, dass jeweils zwei Schlauchabschnitte 2 einer Schlauchmatte 1 mit der jeweils anderen, zu verbindenden Schlauchmatte 1 überlappen. Der Überlappungsbereich B erstreckt sich in Breitenrichtung der jeweiligen Schlauchmatte 1. Es ist auch möglich, den Überlappungsbereich B so zu wählen, dass genau eine oder drei oder mehr Schlauchabschnitte 2 der Schlauchmatten 1 jeweils überlappen.

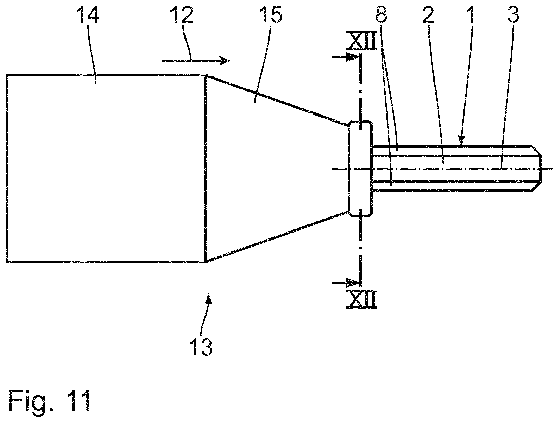

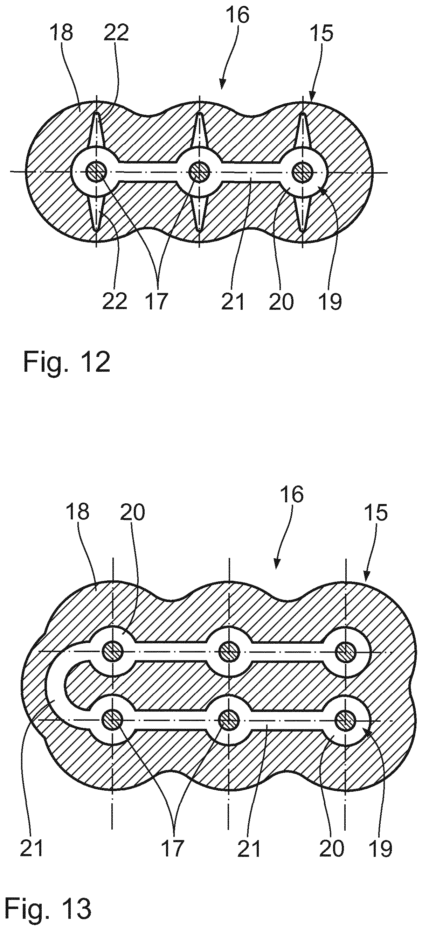

Fig. 11 und 12 zeigen eine Vorrichtung zum Extrudieren einer Schlauchmatte 1 entlang einer Extrusionsrichtung 12. Die Vorrichtung 13 ist in Fig. 11 schematisch dargestellt. Die Vorrichtung 13 umfasst eine Extrusionskammer 14 und eine in Extrusionsrichtung 12 nachgelagerte, mit der Extrusionskammer 14 verbundene Extrusionsdüse 15. Die Extrusionskammer 14 dient zum Aufbereiten einer zu extrudierenden Kunststoffmasse. Zum Aufbereiten einer thermoplastischen Kunststoffmasse dient ein Extruder oder eine Extruderschnecke zum Aufbereiten von Kunststoffgranulat. Dies erfolgt durch Beheizen und/oder Mischen des Granulats, um dasselbe zu schmelzen. Die Kunststoffschmelze wird dann der Extrusionsdüse 15 zugeführt und durch diese die Schlauchmatte 1 entlang der Extrusionsrichtung 12 extrudiert.

Für die Verarbeitung von Elastomeren wie beispielsweise Silikon wird zunächst eine plastische Rohmasse bereit gestellt, die Monomere und Vernetzer wie beispielsweise einen Katalysator oder einen Radikalstarter umfasst. Diese Rohmasse wird kalt, also beispielsweise bei Raumtemperatur, mittels eines Schneckenförderers der Extrusionsdüse 15 zugeführt und durch diese die Schlauchmatte 1 entlang der Extrusionsrichtung 12 extrudiert. Nach dieser Formgebung wird die Schlauchmatte 1 erwärmt, so dass ein thermisches Vernetzen der Monomere erfolgt.

Gemäß dem gezeigten Ausführungsbeispiel weist eine mit der Extrusionsdüse 15 herstellbare Schlauchmatte 1 drei Schlauchabschnitte und zwei dazwischen angeordnete Stegabschnitte auf. Jeweils an einer Ober- und Unterseite der Schlauchabschnitte können Abstandshalter einstückig angeformt werden. Ein gleichzeitiges Extrudieren mehrerer Schlauchabschnitte und Stegabschnitte zu der Schlauchmatte 1 wird durch das in Fig. 12 im Einzelnen dargestellte Werkzeug 16 ermöglicht. Das Werkzeug 16 umfasst neben der Extrusionsdüse 15 mehrere Dorne 17. Die Extrusionsdüse 15 umfasst ein massives Gehäuse 18, in dem eine Mattenkavität 19 vorgesehen ist. Die Mattenkavität 19 umfasst drei, jeweils eine Längsachse aufweisende, parallel zur Extrusionsrichtung 12 angeordnete Schlauchkavität 20 und zwei, jeweils zwischen zwei Schlauchkavitäten 20 angeordnete, die Schlauchkavitäten 20 verbindende Stegkavitäten 21. Koaxial zu der jeweiligen Längsachse einer Schlauchkavität 20 ist der Dorn 17 in der Schlauchkavität 20 angeordnet. Weiterhin sind an den Schlauchkavitäten 20 jeweils zwei Abstandshalterkavitäten 22 vorgesehen, um die Abstandshalter an der Schlauchmatte 1 ausbilden zu können.

Für die Herstellung einer Schlauchmatte 1 gemäß dem ersten Ausführungsbeispiel mit Abstandshaltern derart, die entlang der Schlauchlängsachse 3 eine reduzierte Länge L_{Ab} aufweisen, ist eine zusätzliche, in Fig. 11 und 12 nicht dargestellte Matrix erforderlich. Diese Matrix weist eine Mattenkavität auf, die im Wesentlichen der Mattenkavität 19 der Extrusionsdüse 15 entspricht. Im Bereich der Schlauchkavitäten weist die Mattenkavität der zusätzlichen Matrize allerdings keine Abstandshalterkavitäten auf. Die zusätzliche Matrize ist entlang der Extrusionsrichtung 12 der Extrusionsdüse 15 nachgelagert. Die zusätzliche Matrize ist entlang der Extrusionsrichtung 12 relativ zu der Vorrichtung 13 und insbesondere relativ zu dem Werkzeug 16 verlagerbar. Ein derartiges Verfahren für die Extrusion von Schläuchen mit veränderlichem Außendurchmesser ist aus der US 5,511,965 bekannt.

Fig. 13 zeigt eine weitere Ausführung eines Werkzeugs 16, das im Wesentlichen identisch ist mit dem Werkzeug gemäß Fig. 12. Wesentlicher Unterschied ist die Anzahl der Schlauchkavitäten 20. Insgesamt weist das Werkzeug 16 gemäß Fig. 13 sechs Schlauchkavitäten 20 auf, die in zwei Dreierreihen übereinander in dem Werkzeug 16 angeordnet sind. Dadurch kann vermieden werden, dass das Werkzeug 16 sehr flach und breit ausgeführt ist. Das Werkzeug 16 ist sehr kompakt aufgebaut. Die zwei Dreierreihen der Schlauchkavitäten 20 sind durch eine Stegkavität 21 miteinander verbunden, wobei die verbindende Stegkavität 21 gekrümmt ausgeführt ist. Die verbindenden Stegkavität 21 weist eine Bogenlänge entlang der Krümmung derart auf, dass die Länge identisch ist mit der der übrigen Stegkavitäten 21, die zwischen den jeweils in einer Reihe angeordneten Schlauchkavitäten 20 vorgesehen sind. Aufgrund der elastischen, flexiblen Materialeigenschaften des Silikonkautschuks kann die mit dem Werkzeug 16 in Fig. 13 extrudierte Schlauchmatte 1 flächig in einer Mattenebene 5 angeordnet werden.

Durch die kompakte Ausführung des Werkzeugs 16 ist es möglich, einen erforderlichen Betriebsdruck für die Kunststoffschmelze bzw. die Silikonrohmasse und damit die Werkzeugschließkräfte bei der Herstellung einer Schlauchmatte 1 zu reduzieren. Gleichzeitig ist es möglich, besonders große Schlauchmatten 1, d. h. mit einer großen Anzahl von nebeneinander angeordneten Schlauchabschnitten, effektiv und vorteilhaft herzustellen.

## Patentansprüche

1. Schlauchmatte umfassend
a. mehrere, jeweils eine Schlauchlängsachse (3) aufweisende Schlauchabschnitte (2) und
b. mindestens einen zwei Schlauchabschnitte (2) verbindenden Stegabschnitt (4),
c. mindestens einen Abstandshalter (6; 8; 9), der eine senkrecht zu einer Mattenebene (5) orientierte Dicke (d_{Ab}) aufweist, die größer ist als ein Außendurchmesser (dₐ) eines Schlauchabschnitts (2),
wobei die Schlauchmatte (1) einstückig hergestellt ist,
**dadurch gekennzeichnet, dass**
der Abstandshalter (6; 8; 9) an einem Stegabschnitt (4) und zwischen zwei benachbarten Schlauchabschnitten (2) angeordnet ist, und die Schlauchmatte aufrollbar ist.

2. Schlauchmatte gemäß Anspruch 1, **dadurch gekennzeichnet, dass** der Abstandshalter (6; 8; 9) an einem Schlauchabschnitt (2) angeordnet ist.

3. Schlauchmatte gemäß einem der vorstehenden Ansprüche, **dadurch gekennzeichnet, dass** der Abstandshalter (6; 8; 9) entlang der Schlauchlängsachse (3) eine Länge (L_{Ab}) aufweist, die kleiner ist als eine Länge (Ls) der Schlauchabschnitte (2).

4. Schlauchmatte gemäß Anspruch 3, **gekennzeichnet durch** mehrere Abstandshalter (6; 8; 9) entlang der Schlauchlängsachse (3).

5. Schlauchmatte gemäß Anspruch 1 oder 2, **dadurch gekennzeichnet, dass** der Abstandshalter (6; 8; 9) entlang der Schlauchlängsachse (3) eine Länge (L_{Ab}) aufweist, die im Wesentlichen gleich groß ist wie eine Länge (Ls) der Schlauchabschnitte (2).

6. Schlauchmatte gemäß einem der vorstehenden Ansprüche, **dadurch gekennzeichnet, dass** der Abstandshalter (6; 8; 9) eine senkrecht zur Schlauchlängsachse (3) orientierte Querschnittsfläche aufweist, die eine kreisförmige oder sternförmige Kontur aufweist.

7. Schlauchmatte gemäß einem der vorstehenden Ansprüche, **gekennzeichnet durch** mindestens eine in einem Stegabschnitt (4) angeordnete Ausnehmung (7).

8. Schlauchmatte gemäß einem der vorstehenden Ansprüche, **dadurch gekennzeichnet, dass** die Schlauchmatte (1) aus Silikonkautschuk oder Polyurethan hergestellt ist.

9. Verfahren zum Herstellen einer Schlauchmatte gemäß einem der vorstehenden Ansprüche umfassend die Verfahrensschritte
- Bereitstellen eines Werkzeugs zum Extrudieren einer Schlauchmatte (1) entlang einer Extrusionsrichtung (12),
- Extrudieren einer Schlauchmatte (1) mit mehreren entlang der Extrusionsrichtung (12) angeordneten Schlauchabschnitten (2) und mindestens einem zwei Schlauchabschnitte (2) verbindenden Stegabschnitt (4).

## Claims

1. Tube mat comprising
a. a plurality of tube portions (2) in each case having a tube longitudinal axis (3) and
b. at least one web portion (4) connecting two tube portions (2),
c. at least one spacer (6; 8; 9), which has a thickness (d_{Ab}), which is oriented perpendicular to a mat plane (5) and is greater than an external diameter (dₐ) of a tube portion (2),
wherein the tube mat (1) is produced in one piece,
**characterised in that** the spacer (6; 8; 9) is arranged on a web portion (4) and between two adjacent tube portions (2), and the tube mat is configured to be rolled up.

2. Tube mat according to claim 1, **characterised in that** the spacer (6; 8; 9) is arranged on a tube portion (2).

3. Tube mat according to any one of the preceding claims, **characterised in that** the spacer (6; 8; 9), along the tube longitudinal axis (3), has a length (L_{Ab}), which is smaller than a length (Ls) of the tube portions (2).

4. Tube mat according to claim 3, **characterised by** a plurality of spacers (6; 8; 9) along the tube longitudinal axis (3).

5. Tube mat according to claim 1 or 2, **characterised in that** the spacer (6; 8; 9), along the tube longitudinal axis (3), has a length (L_{Ab}), which is substantially the same size as a length (Ls) of the tube portions (2).

6. Tube mat according to any one of the preceding claims, **characterised in that** the spacer (6; 8; 9) has a cross sectional face, which is oriented perpendicular to the tube longitudinal axis (3) and has a circular or star-shaped contour.

7. Tube mat according to any one of the preceding claims, **characterised by** at least one recess (7) arranged in a web portion (4).

8. Tube mat according to any one of the preceding claims, **characterised in that** the tube mat (1) is produced from silicone rubber or polyurethane.

9. Method for producing a tube mat according to any one of the preceding claims, the method comprising the method steps
- providing a tool for extruding a tube mat (1) along an extrusion direction (12),
- extruding a tube mat (1) with a plurality of tube portions (2) arranged along the extrusion direction (12) and at least one web portion (4) connecting two tube portions (2).

## Revendications

1. Mat tubulaire comprenant
a. plusieurs sections tubulaires (2) présentant chacune un axe longitudinal de mat (3) et
b. au moins une section jointive (4) reliant deux sections tubulaires (2),
c. au moins une entretoise (6 ; 8 ; 9) qui présente une épaisseur (d_{Ab}) orientée perpendiculairement à un plan de mat (5) et supérieure à un diamètre extérieur (dₐ) d'une section tubulaire (2),
dans lequel le mat tubulaire (1) est fabriqué d'une seule pièce,
**caractérisé en ce que**
l'entretoise (6 ; 8 ; 9) est agencée sur une section jointive (4) et entre deux sections tubulaires (2) voisines, et le mat tubulaire est enroulable.

2. Mat tubulaire selon la revendication 1, **caractérisé en ce que** l'entretoise (6 ; 8 ; 9) est agencée sur une section tubulaire (2).

3. Mat tubulaire selon l'une quelconque des revendications précédentes, **caractérisé en ce que** l'entretoise (6 ; 8 ; 9) présente le long de l'axe longitudinal tubulaire (3) une longueur (L_{Ab}) qui est inférieure à une longueur (L_{S}) des sections tubulaires (2).

4. Mat tubulaire selon la revendication 3, **caractérisé par** plusieurs entretoises (6 ; 8 ; 9) le long de l'axe longitudinal tubulaire (3).

5. Mat tubulaire selon la revendication 1 ou 2, **caractérisé en ce que** l'entretoise (6 ; 8 ; 9) présente le long de l'axe longitudinal tubulaire (3) une longueur (L_{Ab}) qui est sensiblement égale à une longueur (L_{S}) des sections tubulaires (2).

6. Mat tubulaire selon l'une quelconque des revendications précédentes, **caractérisé en ce que** l'entretoise (6 ; 8 ; 9) présente une surface de section transversale qui est orientée perpendiculairement à l'axe longitudinal tubulaire (3) qui présente un contour circulaire ou en forme d'étoile.

7. Mat tubulaire selon l'une quelconque des revendications précédentes, **caractérisé par** au moins un évidement (7) ménagé dans une section jointive (4).

8. Mat tubulaire selon l'une quelconque des revendications précédentes, **caractérisé en ce que** le mat tubulaire (1) est fabriqué à partir de caoutchouc de silicone ou de polyuréthane.

9. Procédé de fabrication d'un mat tubulaire selon l'une quelconque des revendications précédentes, comprenant les étapes de procédé suivantes
- fournir un outil pour l'extrusion d'un mat tubulaire (1) le long d'une direction d'extrusion (12),
- extruder un mat tubulaire (1) comprenant plusieurs sections tubulaires (2) agencées le long de la direction d'extrusion (12) et au moins une section jointive (4) reliant deux sections tubulaires (2).
